# EUROPÄISCHE PATENTSCHRIFT

(11) **EP 3 419 566 B1**
(45) Veröffentlichungstag und Bekanntmachung des Hinweises auf die Patenterteilung: **10.09.2025**
(21) Anmeldenummer: 17706807.9
(22) Anmeldetag: 23.02.2017
(51) Int. Cl.: A61F 2/50, A61F 2/78, A61F 2/80

(54) **VERFAHREN ZUM ABFORMEN EINES AMPUTATIONSSTUMPFES UND ABFORMHILFE FÜR EIN SOLCHES VERFAHREN**
METHOD AND DEVICE FOR CASTING A STUMP
MÉTHODE ET DISPOSITIF POUR MODELER UN MOIGNON

(30) Priorität: 23.02.2016 DE 202016001130 U
(43) Veröffentlichungstag der Anmeldung: 02.01.2019
(73) Patentinhaber: Ottobock SE & Co. KGaA, 37115 Duderstadt (DE)
(72) Erfinder: MOSLER, Lüder, 37115 Duderstadt (DE)
(74) Vertreter: Gramm, Lins & Partner Patent- und Rechtsanwälte PartGmbB
(86) Internationale Anmeldenummer: PCT/EP2017/054221
(87) Internationale Veröffentlichungsnummer: WO 2017/144604

(56) Entgegenhaltungen:
- EP-A1- 0 765 646
- WO-A1-2014/032815
- GB-A- 2 188 846
- US-A- 1 335 475
- US-A- 5 432 703

## Beschreibung

Die Erfindung betrifft ein Verfahren zum Abformen eines Amputationsstumpfes, wobei bei dem Verfahren der Amputationsstumpf in einen Liner einer Abformhilfe eingeführt wird, wobei der Liner eine Längsrichtung und einen Umfang aufweist. Die Erfindung betrifft zudem eine Abformhilfe für ein derartiges Verfahren.

Der Prothesenschaft übernimmt in der Prothetik der unteren Extremität die entscheidende Rolle der Schnittstelle zum Körper. Dementsprechend ist das Abformen des Amputationsstumpfes, der auch Prothesenstumpf genannt wird, für die spätere Passform der Prothese ein entscheidender Vorgang.

In der heutigen Praxis dominiert trotz Fortschritten bei den berührungslosen Abformmethoden (Scannen) das Abformen des Stumpfes mittels Gips zur Erstellung einer Negativform des Stumpfes. Neben den geringen Kosten hat diese Methode den Vorteil, dass geübte Orthopädietechniker über eine gezielte Formung des Stumpfes beim Gipsen bereits Einfluss auf die spätere Schaftform nehmen können. Dies gilt insbesondere dann, wenn der spätere Schaft spezifische Belastungszonen aufweisen soll. Dieser Vorteil entfällt, wenn der Schaft vollflächig tragen soll oder wenig erfahrene Techniker das Abformen vornehmen. Deshalb hat sich vermehrtes Interesse an Methoden entwickelt, die während des Abformens gleichmäßigen Druck auf den Stumpf ausüben. Hierzu gehört die von der Firma Össur propagierte Abformung mittels einer aufgepumpten Blase, welche in der EP 0 954 258 gelehrt wird, die von der Northwestern University in Chicago entwickelte Sand-Abform-Methode (Prosthetics and Orthotics International, March 2009; 33(1): 1-9), sowie die von der Universität Strathclyde entwickelte Abformung in einer flexiblen Membran in einem Wassertank (https://givenlimb.org/improved-casting-method-for-leg-prosthetics/). Die letzten zwei Methoden haben hierbei den zusätzlichen Vorteil, dass der Stumpf während des Abformvorganges belastet werden kann, was eine belastungsgerechte Schaftform befördert und dem Nutzer bereits beim Abformvorgang einen Eindruck vom Tragverhalten geben kann.

Aus der WO 2008/092617 A1 ist eine Abformhilfe bekannt, die eine Mehrzahl von Formteilen aufweist, die verschiebbar und verdrehbar angeordnet sind. Dadurch soll die Form des Stumpfes abgenommen werden können. Nachteilig ist jedoch, dass dies nicht unter Belastung des Stumpfes geschehen kann und die Form auch nicht durch optische Verfahren erfassbar ist. Von der Anmelderin wurde das System "anatomical SIT-Cast" verwendet, bei dem die Form des Stumpfes durch eine Abformhilfe abgenommen wurde, die einen Ring aufweist, in den der Stumpf eingeführt wird. Zuvor wird der Stumpf mit einer Gipsmasse und einer Gummimanschette überzogen. Die Erfahrung zeigt, dass dadurch eine nicht optimale Belastung des Stumpfes hervorgerufen wird.

Nachteilig speziell an den zwei letztgenannten Verfahren ist, dass sie bauartbedingt wenig transportabel sind und somit im klinischen Alltag wenig Nutzen bringen. Bei allen drei Verfahren ist es nicht möglich, händisch auf das Abformergebnis Einfluss auszuüben. Eine Kombination mit optischen Scanverfahren, die zunehmend in der modernen Prothetik Verwendung finden, ist ebenfalls nicht möglich.

Aus der WO 2016/135320 A1 ist eine Vorrichtung zum Erstellen eines Gipsabdruckes eines Körpergliedstumpfes bekannt, die einen Druckbehälter ausweist, in den ein vorzugsweise mit Gips eingekleideter Stumpf eingeführt wird. Durch das Fluid soll dann ein gleichmäßiger Druck auf den Stumpf wirken. Nachteilig ist jedoch, dass ein nachträgliches Verformen des Gipses oder des Stumpfes nicht möglich ist und der Stumpf nicht optisch erfasst und vermessen werden kann.

Die US 7,410,350 B2 zeigt eine Vorrichtung, bei der ein eingegipster Amputationsstumpf in einen Behälter eingeführt wird, so dass Expanderelemente, die in dem Behälter angeordnet sind, einen Druck auf den Amputationsstumpf ausüben. Damit sollen die Nachteile einer Lösung behoben werden, bei der ein mit Gips ummantelter Stumpf nach einer ersten Aushärtephase, in der es nicht zur vollständigen Aushärtung kommt, in einem Ring gehalten und von dem Patienten belastet wird.

Aus der US 1 335 475 A und der US 5,432,703 sowie der GB 2 188 846 A sind Verfahren und Vorrichtungen bekannt, die zur Herstellung eines Gipsabdruckes eines Amputationsstumpfes oder zur Herstellung eines festen Objektes, beispielsweise eines Prothesenschaftes verwendet werden. Die EP 0 765 646 A1 beschreibt einen Prothesenliner aus Silikon. Die WO 2014/032815 A1 beschreibt ebenfalls einen Prothesenliner, bei dem verhindert werden soll, dass beim langen Tragen des Liners in einem Rahmenschaft ein Teil des Amputationsstumpfes aus dem Fenster des Rahmenschaftes herausquillt.

Der vorliegenden Erfindung liegt folglich die Aufgabe zugrunde, ein Verfahren zum Abformen eines Amputationsstumpfes und einer Abformhilfe für Prothesenstümpfe zu erstellen, die während des Abformvorganges einen gleichförmigen Druck auf den Stumpf ausüben, vorzugsweise während des Abformvorganges vom Nutzer belastet werden können, die leicht zu handhaben und transportieren sind und einen direkten Zugang zur Oberfläche zwecks Nachformung während des Abformvorganges, oder zum optischen Scannen der Oberflächentopografie anstelle des physischen Abformens ermöglichen.

Die Erfindung löst die gestellte Aufgabe durch ein Verfahren zum Abformen eines Amputationsstumpfes, wobei bei dem Verfahren der Amputationsstumpf in einen Liner einer Abformhilfe eingeführt wird, wobei der Liner eine Längsrichtung und einen Umfang aufweist, wobei sich das Verfahren dadurch auszeichnet, dass der Liner eine Dehnungskopplung derart aufweist, dass eine Verlängerung des Liners entlang der Längsrichtung zwangsläufig eine Verringerung des Umfanges zur Folge hat.

Ein herkömmlicher Liner oder eine aus dem Stand der Technik bekannte elastische Membran bestehen in der Regel aus einem elastischen Material. Befindet sich der Amputationsstumpf in einem derartigen Material und wird beispielsweise von oben in Richtung auf sein distales Ende belastet, führt dies zu einer elastischen Verformung des Liners oder der Membran, die hauptsächlich in einer Verlängerung entlang der Längsachse besteht. Das bedeutet, dass der Liner oder die Membran in Längsachse gedehnt wird. Dies ist bei einem elastischen Material möglich, ohne dass radiale Kräfte auftreten. Im Unterscheid dazu sieht der Liner gemäß dem Abformverfahren der vorliegenden Erfindung vor, dass eine Verlängerung des Liners entlang seiner Längsrichtung zwangsläufig eine Verringerung seines Umfanges zur Folge hat. Wird folglich der Amputationsstumpf im Liner belastet, eine Verlängerung des Liners entlang der Längsrichtung und damit auch eine Verringerung des Umfanges, hat also eine radial auf den Stumpf wirkende Kraft zur Folge. Durch die spezielle Ausgestaltung des Liners wird folglich eine homogene und vollflächige Belastung des Amputationsstumpfes in radialer Richtung erreicht.

Vorzugsweise wird der Amputationsstumpf nach dem Einführen in den Liner durch den Patienten belastet. Dabei kommt es durch die Belastung zu einer Verlängerung des Liners, die durch die besondere Ausgestaltung des Liners eine Verringerung des Umfanges und damit eine radial nach inne wirkende Kraft auf den Stumpf zur Folge hat. Dadurch wird eine sehr gute Belastungssituation des Stumpfes während des Abformvorganges erreicht.

Vorzugsweise wird der Amputationsstumpf vor dem Einführen in den Liner mit einem Abformmaterial, beispielsweise Gips, und vorzugsweise einer Trennschicht, insbesondere einer Trennfolie, eingekleidet. Der Abformvorgang erfolgt dann, indem der Nutzer mit dem Abformmaterial und ggf. der Trennfolie eingekleideten Stumpf in den Liner setzt und durch eine Belastung Zug auf das distale Ende des Liners ausübt, so dass sich der Liner verlängert. Das Setzen oder Einsetzen des Stumpfes oder Amputationsstumpfes in den Liner kann auch als Einführen bezeichnet werden.

Durch die auf das Linerende ausgeübte Zugkraft und die Kopplung der Dehnungen des Liners umschließt nun der Liner den Amputationsstumpf vollständig und übt einen Druck in Umfangsrichtung auf den Stumpf aus, der vorteilhafterweise proportional zur Längsbelastung des Liners ist.

Alternativ oder zusätzlich dazu kann der Amputationsstumpf nach dem Einführen in den Liner optisch vermessen werden. Dabei wird der Amputationsstumpf vorzugsweise durch den Patienten belastet. Auch hier handelt es sich bei der bereits beschriebenen Belastung, die Zug auf das distale Linerende ausübt und somit einen Druck in Umfangsrichtung auf den Amputationsstumpf erzeugt.

Vorzugsweise wird nach dem Einführen des Amputationsstumpfes in den Liner der Amputationsstumpf und/oder das Abformmaterial verformt, um die bereits beschriebenen vorteilhaften Wirkungen zu erreichen.

Die Erfindung löst die gestellte Aufgabe zudem durch eine Abformhilfe für ein hier beschriebenes Verfahren. Diese folglich über den hier beschriebenen Liner mit der beschriebenen Dehnungskopplung, der in einer Halteeinrichtung gehalten wird, die höhenverstellbar ist. Der Liner weist vorteilhafterweise einen Flechtschlauch oder ein Netz aus vorzugsweise sich kreuzenden Fasern auf oder besteht da-raus. Die Fasern verlaufen in einem unbelasteten Zustand des Liners vorteilhafterweise in einem Winkel von 45° mit der Längsrichtung des Liners. Man spricht von einem diagonalen Faserverlauf. Dabei ist ein exakter Winkel von 45° nicht notwendig, solange die gewünschte Kopplung zwischen der Längsdehnung und der Verringerung des Umfanges erreicht wird. Über den Winkel zwischen dem Faserverlauf und der Längsrichtung des Liners kann die auf den Amputationsstumpf ausgeübte Kraft, die durch eine Verlängerung des Liners entlang seiner Längsrichtung hervorgerufen wird, eingestellt werden. Je flacher die Fasern verlaufen, also je näher der beschriebene Winkel einem rechten Winkel kommt, desto größer ist die bei Belastung entlang der Längsrichtung auftretende Kraft in Radialrichtung.

Vorteilhafterweise wird der Liner in einem Befestigungsring gehalten. Der Liner wird dabei in einer bevorzugten Ausgestaltung an seinem proximalen Ende ringförmig gehalten.

Um eine möglichst freie Orientierung des Amputationsstumpfes zu ermöglichen, kann die Halteeinrichtung, also insbesondere der proximale Befestigungsring, so gelagert sein, dass eine gleichmäßige Längskraft am gesamten Umfang des Liners auftritt. Hierzu sind verschiedene Lösungen denkbar:
- Der Befestigungsring kann kardanisch gelagert sein.
- Der Befestigungsring kann eine sphärische Auflage besitzen, die in einem entsprechend geformten Ring gelagert ist.
- Der Befestigungsring kann an mindestens zwei angewinkelten Lenkern oder Blattfedern gelagert sein, die aufgrund ihrer Mehrgelenkskinematik ein Verschwenken des Befestigungsringes ermöglichen.
- Der Befestigungsring kann ein Straffen des Liners im Anpassungsvorgang so ermöglichen, dass der Liner sich entsprechend der Orientierung des Stumpfes ausrichten kann und entsprechend der Fixierung am proximalen Rand der Orientierung des Stumpfes folgt.

Erfindungsgemäß ist die Halteeinrichtung höhenverstellbar ausgebildet. Dadurch kann auf unterschiedlich große Patienten und unterschiedlich lange Amputationsstümpfe auf besonders einfache Weise eingegangen werden.

Hierzu ist vorteilhafterweise eine variable Klemmvorrichtung für den proximalen Rand des Liners vorgesehen. In dieser bevorzugten, besonders einfachen Lösung kann der Liner am proximalen Ende um einen losen Ring umgeschlagen sein, um dann innerhalb des Befestigungsringes in Richtung distal geführt zu werden. Durch Zug am Linerrand Richtung distal kann der Liner am Stumpf zur Anlage gebracht werden, während der Nutzer einbeinig steht. Durch die zweifache Umlenkung des Liners wir dieser im Befestigungsring fixiert und ist vom Benutzer belastbar.

Vorzugsweise ist der Liner um einen Ring umgelenkt und auf sich selbst zurückgeführt, wobei der Ring mit dem Liner zur Auflage einer Halteeinrichtung kommt. Diese besonders einfache Art der Befestigung des Liners ermöglicht eine konstruktiv einfache und damit kostengünstig herzustellende Abformhilfe. Die Halteeinrichtung ist vorteilhafterweise an einer Stützeinrichtung, beispielsweise einer Säule, befestigt und vorteilhafterweise schwenkbar angeordnet. Dadurch kann die Halteeinrichtung vorteilhafterweise um die Längseinrichtung der Stützeinrichtung verschwenkt werden.

In einer bevorzugten Ausgestaltung ist die Halteeinrichtung klappbar an der Stützeinrichtung angeordnet. Dadurch ist sie in eine Verwendungsposition, in der der Patient in einen in der Halteeinrichtung angeordneten Liner einsteigen und seinen Amputationsstumpf in den Liner einführen kann, und in eine Transportstellung bringbar, in der die Halteeinrichtung beispielsweise an die Stützeinrichtung angeklappt ist, um den benötigten Bauraum zu verringern. Vorteilhafterweise greift die Halteeinrichtung insbesondere in einer dieser Stellungen, vorteilhafterweise in einer heruntergeklappten Stellung, die der Benutzungsstellung entspricht, in Rastelemente, vorzugsweise Rasten, der Stützeinrichtung ein oder verklemmt oder verkeilt sich mit der Stützeinrichtung. Dadurch wird eine ausreichende Stabilität erreicht und eine Fehlbedienung nahezu ausgeschlossen.

Die Stützeinrichtung, also insbesondere der Befestigungsring, sollte so angebracht sein, dass für einen Orthopädietechniker ein guter Zugang möglich ist. Gleichzeitig ist die Stützeinrichtung vorteilhafterweise höhenverstellbar und auswechselbar. Auf diese Weise können beispielsweise unterschiedliche Größen eines Befestigungsringes verwendet werden. Um einen leichten Transport zu ermöglichen, ist es hilfreich, wenn die gesamte Abformhilfe zusammenklappbar ist, um beispielsweise in einer Werkstatt einfach verstaubar und im klinischen Alltag transportierbar zu sein. In einer bevorzugten Ausgestaltung der Erfindung ist daher die Halteeinrichtung, insbesondere der Befestigungsring, klappbar an einer vertikalen Säule befestigt. Hierzu können beispielsweise ein Scharnier und eine feststellbare Schelle um die Säule vorgesehen sein. In einer bevorzugten Lösung erzeugt die Halteeinrichtung die Kräfte zu seiner Arretierung selbst, indem der Befestigungsring belastet wird. Hierzu kann der Befestigungsring beim Herunterklappen beispielsweise in Rasten in der Säule eingreifen. Bei entsprechender Auslegung ist es auch möglich, die Arretierung nur durch Reibkräfte zu erzeugen, was eine stufenlose Höhenverstellung ermöglicht. Ebenso kann die Säule der Abformhilfe so gestaltet sein, dass sie für die Lagerung und den Transport aus ihrer Befestigung beispielsweise auf einer Bodenplatte gelöst werden kann. Hierzu kann eine lösbare Verbindung, beispielsweise eine Steckverbindung vorgesehen sein. In einer weiteren, bevorzugten Ausführung ist die Säule über ein Scharnier mit der Bodenplatte verbunden. Sie kann in die gewünschte Stellung geschwenkt werden. Vorzugsweise bildet die Arbeitsstellung der Säule den Anschlag einer derartigen Schwenkbewegung, so dass die Säule beim Abformvorgang eine stabile, vorzugsweise senkrechte Position einnimmt. Für die Lagerung oder den Transport kann sie beispielsweise gegen die Bodenplatte verschwenkt werden.

Vorzugsweise verfügt der Liner, der insbesondere aus einem Linermaterial hergestellt ist, über mehrere Fasern, die in dem Linermaterial angeordnet sind. Diese Fasern bestehen vorzugsweise aus einem unelastischen Material. Der Liner kann mehrere, vorzugsweise zwei, Faserlagen aufweisen, die eine Mehrzahl von Fasern aufweisen, die vorteilhafterweise zumindest über einen Bereich hinweg jeweils in einer Faserlage parallel zueinander verlaufen. Solange die Fasern der unterschiedlichen Faserlagen einander kreuzen, lässt sich auch auf diese Weise der gewünschte Effekt der Kopplung der Dehnungen erreichen.

Zusätzlich oder alternativ dazu können die Fasern auch eine spiralförmige Matrix bilden. Dabei verfügt der Liner über erste Fasern, die eine spiralförmige Matrix in einer ersten Umlaufrichtung und zweite Fasern, die eine spiralförmige Matrix in einer zweiten Umlaufrichtung, die der ersten Umlaufrichtung entgegengesetzt ist, bilden. Spiralförmig bedeutet in diesem Fall, dass sich die jeweilige Faser in vorzugsweise mehreren Windungen um den Umfang des Amputationsstumpfes und damit des Liners herum erstreckt, wenn der Liner an dem Amputationsstumpf anliegt.

Vorteilhafterweise kreuzen sich die Fasern, die in dem Linermaterial angeordnet sind, in einem rechten Winkel. Dies gilt insbesondere für den Fall, dass sich kein Amputationsstumpf in dem Liner befindet. Durch das Einführen eines Stumpfes und insbesondere die Belastung kann dieser Winkel verschoben werden.

Selbstverständlich kann der Liner auch aus mehreren Teillinern bestehen, die jeweils nur eine Faserlage mit nur einer Faserrichtung aufweisen. Alternativ zu sich kreuzenden Fasern können beispielsweise auch hexagonale Netze aus unelastischen Fasern in das Linermaterial eingebettet werden. Selbstverständlich sind auch andere Netze, die die gewünschten Eigenschaften aufweisen möglich. Dies bedeutet, dass eine Verlängerung des Netzes in einer Richtung zwangsläufig eine Verkürzung des Netzes in der anderen Richtung zur Folge hat.

Nachfolgend werden Ausführungsbeispiele der vorliegenden Erfindung anhand der beigefügten Zeichnungen näher erläutert. Es zeigt:
- Figuren 1 a und b -: schematische Darstellungen eines Liners und eines Amputationsstumpfes vor und nach dem Einführen;
- Figuren 2 a und b -: schematische Zeichnungen zur Wirkungsweise der Festlegung des Liners;
- Figur 3 -: die schematische Darstellung einer Abformhilfe mit eingeführtem Amputationsstumpf;
- Figur 4 -: die schematische Darstellung einer Abformhilfe in Transportstellung;
- Figuren 5 bis 8 -: verschiedene Stadien in einem Verfahren zum Abformen gemäß einem Ausführungsbeispiel der vorliegenden Erfindung und
- Figuren 9 und 10 -: zwei Abbildungen in unterschiedlichen Stadien eines Verfahrens gemäß einem weiteren Ausführungsbeispiel der vorliegenden Erfindung.

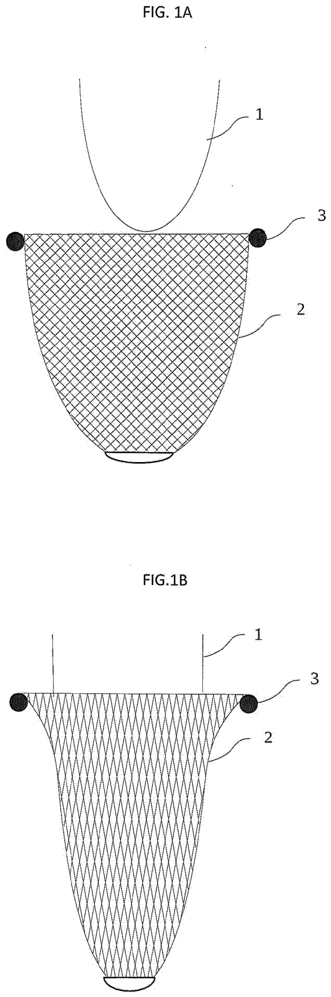

Fig. 1 zeigt schematisch die Funktion der Kopplung von Längs- und Querdehnung im Liner (2), währen der Prothesenstumpf aufgenommen wird. Dieser ist am Befestigungsring (3) befestigt und besteht - wie in der Oberflächentextur angedeutet - aus einem Netz oder Geflecht mit diagonal verlaufender Faserorientierung. Durch Längsverformung des Liners kommt es zu einer verstärkten Orientierung der Fasern in Längsrichtung, was gleichzeitig den Umfang des Liners verringert. Die Umformung des Liners kommt zum Stillstand, wenn der Stumpf (1) vollständig umschlossen ist. Bei einer Belastung des Stumpfes wird ein gleichmäßiger Druck auf den Stumpf ausgeübt. Der Liner besteht aus einem Flechtschlauch aus Garn oder Monofilamenten oder einem Netz in Schlauchform mit einem diagonalem Verlauf der Fäden oder mit einer Hexagon-Struktur. Besonders vorteilhaft sind Flechtschläuche aus Polyamid oder Polypropyen Monofilamenten, da diese sehr beweglich sind und trotzdem eine geschlossene Oberfläche bilden. Die Größe ist so zu wählen, dass der Stumpf vollständig umschlossen wird, wenn die Fäden des Flechtschlauchs etwa 90° Kreuzungswinkel aufweisen, bzw. das hexagonale Netz unverzerrt ist. Distal kann der Schlauch zusammengebunden sein oder mit einer flexiblen Abschlusskappe (9) verklebt oder verschweißt sein. Es bietet sich auf jeden Fall an, innen distal eine glatte Fläche zu erzeugen indem ein elastischer Teller eingeklebt ist oder das distale Ende mit Elastomer vergossen ist. Proximal ist der Liner mit dem Befestigungsring (3) verbunden.

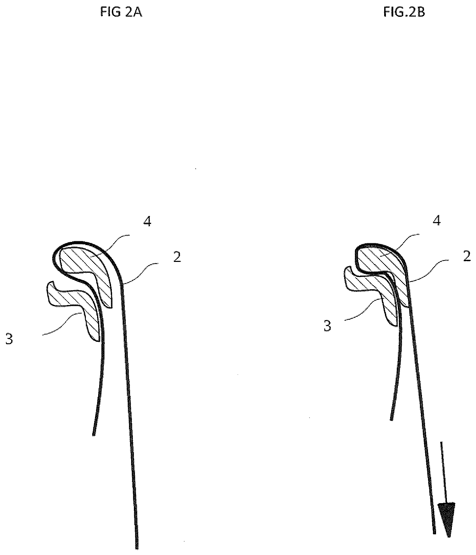

Fig. 2 zeigt eine vorteilhafte Befestigung des Liners am Befestigungsring (3) in der Schnittdarstellung. Der Befestigungsring (3) kann gestufte ausgeführt sein um eine Zentrierung für den Ring (4) zu bieten. Der Liner (2) wird um den Ring (4) geschlagen und innerhalb des Befestigungsrings (3) auf sich selbst zurückgeführt. Durch die mehrfache Umlenkung kommt es bei Belastung des Liners - wie in Fig. 2B dargestellt - zu einem festen Andrücken des Rings (4) auf den Befestigungsring (3) und damit zu hohen Reibkräften, die den Liner sicher im Befestigungsring halten, solange dieser belastet wird. Vorteilhaft an dieser Anordnung ist, dass der Liner innerhalb des Rings entsprechend der Stellung des Unterschenkels orientiert werden kann. Idealerweise kommt es zur Anpassung indem der Liner durch Zug am heruntergeschlagenen Ende am Stumpf gestrafft und anschließend durch den Nutzer belastet wird.

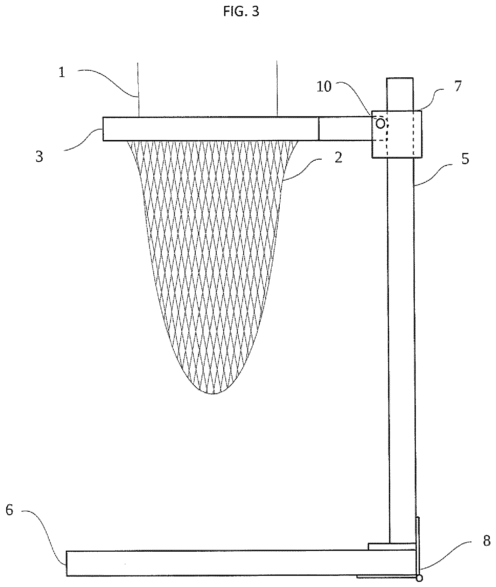

Fig. 3 zeigt eine mögliche Ausführung der gesamten Abformhilfe. Der Liner (2) nimmt den Stumpf (1) auf. Sein proximales Ende ist im Befestigungsring (3) gefasst. Dieser ist mittels einer Schelle (7) an der Säule (5) befestigt. Die Achse (10) erlaubt es, den Befestigungsring (3) an die Säule hochzuklappen. Heruntergeklappt entsteht durch die Formgebung ein Anschlag, der den Befestigungsring in einer waagerechten Stellung hält. Gleichzeitig wirken bei Belastung des Befestigungsringes von Oben starke Kräfte auf die Säule, die ein Verrutschen der Schelle entlang der Säule verhindern. Auf diese Weise ist der Befestigungsring (3) stufenlos höhenverstellbar, ohne dass dafür eine Arretierung gelöst werden müsste. Die Säule (5) ist mittels eines Scharniers an der Bodenplatte (6) befestigt. Durch die Lage dessen Drehpunktes entsteht in senkrechter Position der Säule ein Anschlag, der durch Belastung des Befestigungsringes (3) von Oben stabilisiert wird. Ein Einsatz der Abformhilfe ist somit möglich, ohne die Gefahr, dass die Säule nach Rückwärts verschwenkt.

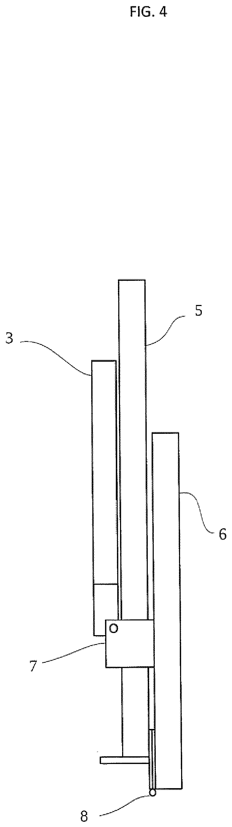

Fig. 4 zeigt die Abformhilfe aus Fig. 3 im zusammengeklappten Zustand. Die Säule (5) ist hierbei auf die Unterseite der Bodenplatte (6) geschwenkt. Der Befestigungsring (3) ist gegen die Säule (5) geklappt. Erkennbar ist die Abformhilfe in diesem Zustand sehr kompakt und kann leicht transportiert und verstaut werden. Der Aufbau erfordert wenige Handgriffe.

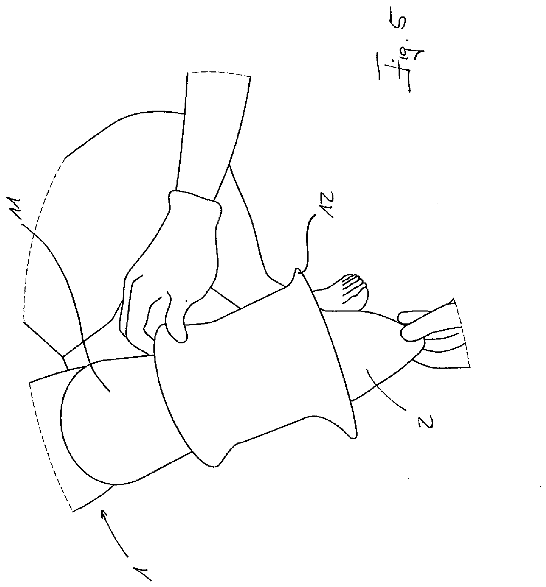

Fig. 5 zeigt wie der Amputationsstumpf 1, der im gezeigten Ausführungsbeispiel mit einer Modellierschicht aus Gips 11 und einer Trennfolie eingekleidet ist, in den Liner 2 eingeführt wird. Dabei ist ein distaler Rand 12 des Liners 2 umgeschlagen. Die einzelnen Fasern, durch die in dem gezeigten Liner 2 die Kopplung zwischen Längsdehnung und Umfangsverringerung erreicht wird, sind in den Figuren 5 bis 10 nicht dargestellt.

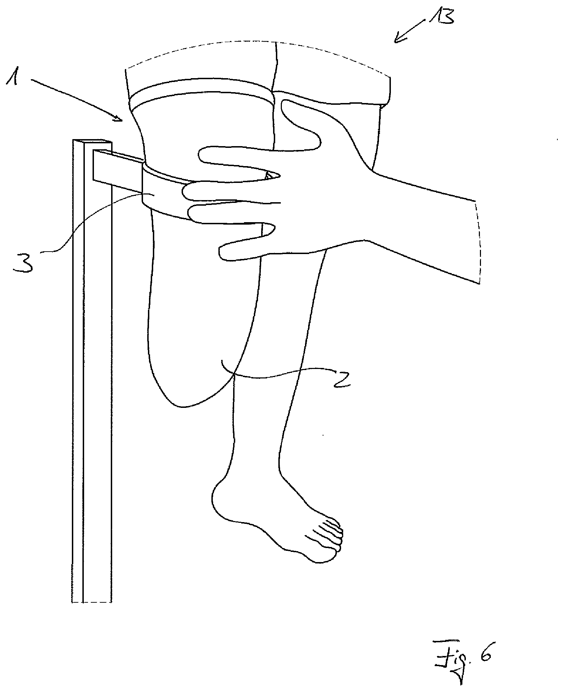

Dies ist in Fig. 6 dargestellt. Der Patient 13 steht auf seinem gesunden Bein und der Stumpf 1 mit dem Liner 2 ist in den Befestigungsring 3 eingeführt und wird dort gehalten. Der Patient 13 kann nun den Amputationsstumpf 1 belasten und sorgt so dafür, dass der Liner 2 in Längsrichtung, in Fig. 6 also von oben nach unten, verlängert wird. Durch die Kopplung der Längs- und Querdehnung führt dies zwangsläufig zu einer Verringerung des Umfanges um somit zu einer auf den Amputationsstumpf 1 wirkenden Kraft, die radial nach innen gerichtet ist.

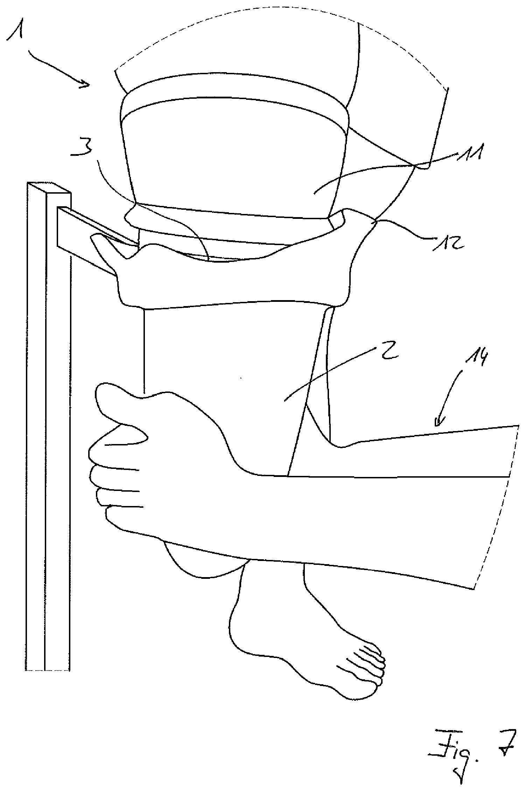

In Fig. 7 ist die Situation aus Fig. 6 dargestellt, wobei nun eine weitere Person, beispielsweise ein Orthopädietechniker 14 den Amputationsstumpf 1 in dem Liner 2 formen kann, während der Amputationsstumpf 1 in dem Befestigungsring 3 gehalten ist. Dadurch kann die Schicht Gips 11, die sich innerhalb des Liners 2 befindet, modelliert und gegebenenfalls an- oder umgeformt werden. Auf diese Weise kann den individuellen Gegebenheiten des Amputationsstumpfes 1 Rechnung getragen werden.

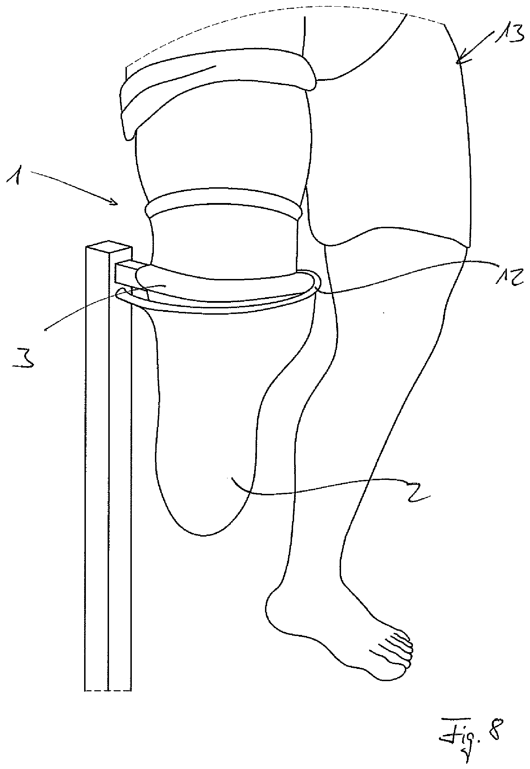

Fig. 8 zeigt die abschließende Phase, in der der Patient 13 auf seinem gesunden Bein steht und den Amputationsstumpf 1 innerhalb des Befestigungsrings 3 belastet. Dadurch wird die bereits beschriebene Kraft durch den Liner 2 auf den Amputationsstumpf und das Formmaterial aus Gips 11 aufgebracht, das sich innerhalb des Liners befindet, bis das Material ausreichend ausgehärtet ist, um den Liner aus dem Befestigungsring 3 zu entfernen.

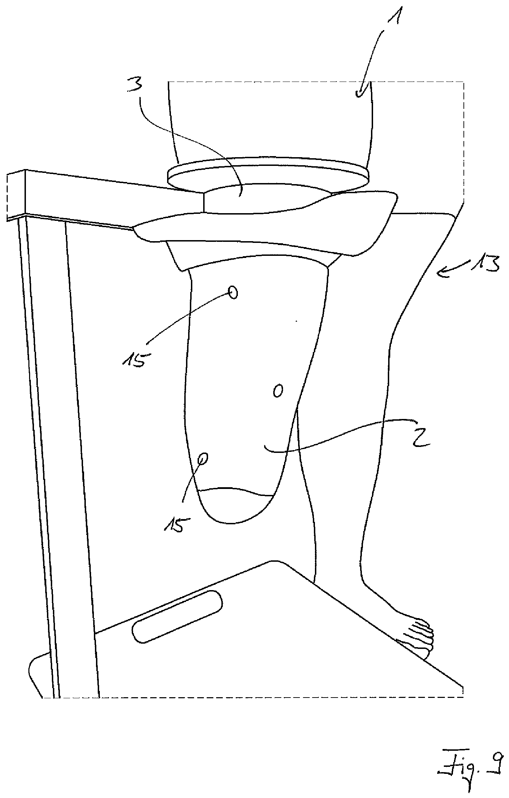

Fig. 9 zeigt die Situation bei einem Verfahren gemäß einem weiteren Ausführungsbeispiel der vorliegenden Erfindung. Der Patient 13 steht wieder auf seinem gesunden Bein und der Amputationsstumpf 1 ist in den Liner 2 eingeführt. Anders als bei den Figuren 5 bis 8 ist jedoch kein Gips 11 innerhalb des Liners zwischen dem Liner 2 und dem Amputationsstumpf 1 angeordnet. Der Liner wird wieder im Befestigungsring 3 gehalten, verfügt nun aber über Markierungen 15, die eine optische Vermessung erlauben.

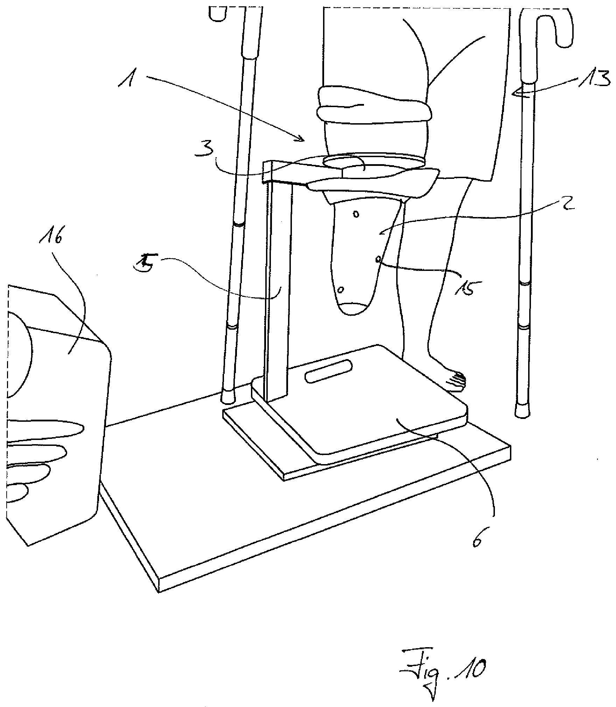

Diese wird in Fig. 10 dargestellt. Der Amputationsstumpf 1 des Patienten 13 befindet sich im Liner 2, der die in Fig. 9 gezeigte Ausgestaltung hat. Mittels eines Lasermessgerätes 16, wie aus dem Stand der Technik prinzipiell bekannt ist, wird nun die geometrische Form des Liners 2, in dem sich der Amputationsstumpf 1 befindet, dreidimensional vermessen. Die so ermittelten Daten können zur Herstellung des Prothesenschaftes verwendet werden. Der Befestigungsring 3 ist an der Säule 5 angeordnet und vorzugsweise höhenverstellbar, also insbesondere entlang der Längsrichtung der Säule verschiebbar. In einer besonders bevorzugten Ausgestaltung ist der Befestigungsring 3 zudem gegen die Säule 5 verschwenkbar, um die Abformhilfe in die in Fig. 5 gezeigte Transportstellung bringen zu können. Dazu ist es von Vorteil, wenn auch die Bodenplatte 6 relativ zur Säule 5 verschwenkbar ist.

### Bezugszeichenliste:

- 1: Stumpf
- 2: Liner
- 3: Befestigungsring
- 4: Ring
- 5: Säule
- 6: Bodenplatte
- 7: Schelle
- 8: Scharnier
- 9: Abschlusskappe
- 10: Achse
- 11: Gips
- 12: distaler Rand
- 13: Patient
- 14: Orthopädietechniker
- 15: Markierung
- 16: Lasermessgerät

## Patentansprüche

1. Verfahren zum Abformen eines Amputationsstumpfes, wobei bei dem Verfahren der Amputationsstumpf in einen Liner (2) einer Abformhilfe eingeführt wird, wobei der Liner (2) eine Längsrichtung und einen Umfang aufweist,
**dadurch gekennzeichnet, dass** der Liner (2) eine Dehnungskopplung derart aufweist, dass eine Verlängerung des Liners (2) entlang der Längsrichtung zwangsläufig eine Verringerung des Umfangs zur Folge hat.

2. Verfahren nach Anspruch 1, **dadurch gekennzeichnet, dass** der Amputationsstumpf nach dem Einführen in den Liner (2) durch den Patienten (13) belastet wird.

3. Verfahren nach Anspruch 1 oder 2, **dadurch gekennzeichnet, dass** der Amputationsstumpf vor dem Einführen in den Liner (2) mit einem Abformmaterial eingekleidet wird.

4. Verfahren nach Anspruch 1, 2 oder 3, **dadurch gekennzeichnet, dass** der Amputationsstumpf nach dem Einführen in den Liner (2) optisch vermessen wird.

5. Verfahren nach einem der vorstehenden Ansprüche, **dadurch gekennzeichnet, dass** nach dem Einführen des Amputationsstumpfes in den Liner (2) der Amputationsstumpf und/oder das Abformmaterial verformt wird.

6. Abformhilfe für ein Verfahren nach einem der vorstehenden Ansprüche, die einen Liner (2) aufweist, der eine Dehnungskopplung derart aufweist, dass eine Verlängerung des Liners (2) entlang der Längsrichtung zwangsläufig eine Verringerung des Umfangs zur Folge hat, **dadurch gekennzeichnet, dass** der Liner (2) in einer Halteeinrichtung gehalten wird, die höhenverstellbar ist.

7. Abformhilfe nach Anspruch 6, **dadurch gekennzeichnet, dass** der Liner (2) einen Flechtschlauch oder ein Netz aus Fasern aufweist oder daraus besteht.

8. Abformhilfe nach Anspruch 7, **dadurch gekennzeichnet, dass** die Fasern in einem unbelasteten Zustand einen Winkel von 45° mit der Längsrichtung des Liners (2) einschließen.

9. Abformhilfe nach einem der Ansprüche 6 bis 8, **dadurch gekennzeichnet, dass** der Liner (2) um einen Ring (4) umgelenkt und auf sich selbst zurückgeführt ist, wobei der Ring (4) mit dem Liner (2) zur Auflage auf der Halteeinrichtung kommt.

10. Abformhilfe nach einem der Ansprüche 6 bis 9, **dadurch gekennzeichnet, dass** die Halteeinrichtung austauschbar oder aufweitbar ist.

11. Abformhilfe nach einem der Ansprüche 6 bis 10, **dadurch gekennzeichnet, dass** die Halteeinrichtung an einer Stützeinrichtung befestigt ist.

12. Abformhilfe nach Anspruch 11, **dadurch gekennzeichnet, dass** die Halteeinrichtung klappbar an der Stützeinrichtung angeordnet ist.

13. Abformhilfe nach Anspruch 12, **dadurch gekennzeichnet, dass** die Halteeinrichtung in einer Stellung in Rastelemente der Stützeinrichtung eingreift oder mit der Stützeinrichtung verklemmt oder verkeilt.

## Claims

1. A method for making a mold of an amputation stump, in which method the amputation stump is inserted into a liner (2) of a molding aid, said liner (2) having a longitudinal direction and a circumference, **characterized in that** the liner (2) has an expansion coupling, such that a lengthening of the liner (2) along the longitudinal direction necessarily results in a reduction of the circumference.

2. The method as claimed in claim 1, **characterized in that** the amputation stump, after insertion into the liner (2), is loaded by the patient (13).

3. The method as claimed in claim 1 or 2, **characterized in that** the amputation stump, prior to insertion into the liner (2), is equipped with a molding material.

4. The method as claimed in claim 1, 2 or 3, **characterized in that** the amputation stump is measured optically after insertion into the liner (2).

5. The method as claimed in one of the preceding claims, **characterized in that**, after insertion of the amputation stump into the liner, the amputation stump and/or the molding material is shaped.

6. A molding aid for a method as claimed in one of the preceding claims, comprising a liner (2), that has an expansion coupling, such that a lengthening of the liner (2) along the longitudinal direction necessarily results in a reduction of the circumference, **characterized in that** the liner (2) is held in a retaining device, which is adjustable in height.

7. The molding aid as claimed in claim 6, **characterized in that** the liner (2) has or is made of a braided tube or a net of fibers.

8. The molding aid as claimed in claim 7, **characterized in that** the fibers, in an unloaded state, enclose an angle of 45° with the longitudinal direction of the liner (2).

9. The molding aid as claimed in one of claims 6 to 8, **characterized in that** the liner (2) is deflected around a ring (4) and guided back onto itself, wherein the ring (4) with the liner (2) comes to bear on the retaining device.

10. The molding aid as claimed in one of claims 6 to 9, **characterized in that** the retaining device is exchangeable or expandable.

11. The molding aid as claimed in one of claims 6 to 10, **characterized in that** the retaining device is secured on a support device.

12. The molding aid as claimed in claim 11, **characterized in that** the retaining device is secured foldably on said support device.

13. The molding aid as claimed in claim 12, **characterized in that** the retaining device in one position engages in latching elements of the support device, or is clamped or wedged onto the support device.

## Revendications

1. Procédé pour modeler un moignon d'amputation, procédé dans lequel le moignon d'amputation est introduit dans une gaine (2) d'un dispositif auxiliaire de modelage, la gaine (2) présentant une direction longitudinale et une circonférence,
**caractérisé en ce que** la gaine (2) présente un couplage d'extension tel qu'un allongement de la gaine (2) le long de la direction longitudinale entraîne nécessairement une réduction de la circonférence.

2. Procédé selon la revendication 1,
**caractérisé en ce que** le moignon d'amputation est mis sous contrainte par le patient (13) après avoir été introduit dans la gaine (2).

3. Procédé selon la revendication 1 ou 2,
**caractérisé en ce que** le moignon d'amputation est revêtu d'un matériau de modelage avant d'être introduit dans la gaine (2).

4. Procédé selon la revendication 1, 2 ou 3,
**caractérisé en ce que** le moignon d'amputation est mesuré optiquement après avoir été introduit dans la gaine (2).

5. Procédé selon l'une des revendications précédentes,
**caractérisé en ce que**, après l'introduction du moignon d'amputation dans la gaine (2), le moignon d'amputation et/ou le matériau de modelage est déformé.

6. Dispositif auxiliaire de modelage pour un procédé selon l'une des revendications précédentes, comprenant une gaine (2) qui présente un couplage d'extension tel qu'un allongement de la gaine (2) le long de la direction longitudinale entraîne nécessairement une réduction de la circonférence,
**caractérisé en ce que** la gaine (2) est maintenue dans un moyen de maintien qui est réglable en hauteur.

7. Dispositif auxiliaire de modelage selon la revendication 6,
**caractérisé en ce que** la gaine (2) comprend ou est constituée d'un tuyau tressé ou d'un filet de fibres.

8. Dispositif auxiliaire de modelage selon la revendication 7,
**caractérisé en ce que** les fibres définissent un angle de 45° avec la direction longitudinale de la gaine (2), à l'état non contraint.

9. Dispositif auxiliaire de modelage selon l'une des revendications 6 à 8, **caractérisé en ce que** la gaine (2) est déviée autour d'un anneau (4) et est ramenée sur elle-même, l'anneau (4) pourvue de la gaine (2) venant s'appuyer sur le moyen de maintien.

10. Dispositif auxiliaire de modelage selon l'une des revendications 6 à 9, **caractérisé en ce que** le moyen de maintien est interchangeable ou expansible.

11. Dispositif auxiliaire de modelage selon l'une des revendications 6 à 10, **caractérisé en ce que** le moyen de maintien est fixé à un organe de support.

12. Dispositif auxiliaire de modelage selon la revendication 11,
**caractérisé en ce que** le moyen de maintien est disposé de manière rabattable sur l'organe de support.

13. Dispositif auxiliaire de modelage selon la revendication 12,
**caractérisé en ce que** dans une position, le moyen de maintien s'engage dans des éléments d'encliquetage de l'organe de support ou se coince ou se bloque avec l'organe de support.
